# EUROPEAN PATENT SPECIFICATION

(11) **EP 3 622 958 B1**
(45) Date of publication and mention of the grant of the patent: **20.03.2024**
(21) Application number: 18798117.0
(22) Date of filing: 08.05.2018
(51) Int. Cl.: A61P 25/24, A61K 31/7105, C12N 15/113

(54) **USE OF POTASSIUM ION CHANNEL INHIBITOR FOR TREATMENT OF DEPRESSION AND PHARMACEUTICAL COMPOSITION**
VERWENDUNG EINES KALIUMIONENKANALHEMMERS ZUR BEHANDLUNG VON DEPRESSION UND PHARMAZEUTISCHE ZUSAMMENSETZUNG
UTILISATION D'UN INHIBITEUR DE CANAL IONIQUE POTASSIQUE POUR LE TRAITEMENT DE LA DÉPRESSION ET COMPOSITION PHARMACEUTIQUE

(30) Priority: 09.05.2017 CN 201710322245
(43) Date of publication of application: 18.03.2020
(73) Proprietor: Zhejiang University, Hangzhou, Zhejiang 310058 (CN)
(72) Inventor: HU, Hailan, Hangzhou Zhejiang 310058 (CN); CUI, Yihui, Hangzhou Zhejiang 310058 (CN); YANG, Yan, Hangzhou Zhejiang 310058 (CN)
(74) Representative: Brevalex
(86) International application number: PCT/CN2018/086021
(87) International publication number: WO 2018/205927

(56) References cited:
- ANDREWS J M ET AL: "Contemporary management of depression", AMERICAN JOURNAL OF MEDICINE, EXCERPTA MEDICA, INC, UNITED STATES, vol. 97, no. 6, 19 December 1994 (1994-12-19), pages S24-S32, XP023307550, ISSN: 0002-9343, DOI: 10.1016/0002-9343(94)90360-3 [retrieved on 1994-12-19]
- OHNO ET AL: "Inhibition of astroglial Kir4.1 channels by selective serotonin reuptake inhibitors", BRAIN RESEARCH, ELSEVIER, AMSTERDAM, NL, vol. 1178, 26 October 2007 (2007-10-26), pages 44-51, XP022317348, ISSN: 0006-8993, DOI: 10.1016/J.BRAINRES.2007.08.018
- S. SU ET AL: "Inhibition of Astroglial Inwardly Rectifying Kir4.1 Channels by a Tricyclic Antidepressant, Nortriptyline", JOURNAL OF PHARMACOLOGY AND EXPERIMENTAL THERAPEUTICS, vol. 320, no. 2, 27 October 2006 (2006-10-27), pages 573-580, XP055547641, US ISSN: 0022-3565, DOI: 10.1124/jpet.106.112094
- LI, FAN: "Role of Inwardly Rectifying Potassium Channel Kir4.1 in Oligodendrocyte Development and Myelin Formation", Master Thesis, 15 March 2017 (2017-03-15), pages 1-65, XP009517920,
- FURUTANI, K. et al.: "2401-Pos Board B371. The Structural Basis for Antidepressants Blok being Confined to Kir4.1", Biophysical Journal, vol. 96, no. 3, 3 March 2009 (2009-03-03), page 465a, XP055547634,
- SU , S. et al.: "Inhibition of Astroglial Inwardly Rectifying Kir4.1 Channels by a Tricyclic Antidepressant, Nortriptyline", Journal of Pharmacology & Experimental Therapeutics, vol. 320, no. 2, 1 February 2007 (2007-02-01), pages 573-580, XP055547641,
- HIGASHI, K et al.: "An Inwardly Rectifying K+ Channel, kir4.1, Expressed in Astrocytes Surrounds Synapses and Blood Vessels in Brain", American Journal of Physiology-Cell Physiology, vol. 281, no. 3, 30 September 2001 (2001-09-30), pages C922-C931, XP055547655,

## Description

### TECHNICAL FIELD

The disclosure relates to the field of disease therapy and pharmacy field, and in particular, provides pharmaceutical compositions for treating depression.

### BACKGROUND

Depression is a chronic mental disorder characterized by significant and persistent low mood, lack of motivation, behavioral despair, and loss of pleasure. Patients with depression may even show suicidal tendency.

Lateral habenula (LHb) has been considered as a key brain area for studying the pathophysiology of depression in recent years. Significant elevation of lateral habenular neurons activity has been found in many animal models of depression and patient of depression.

Abnormal neuron activities are mainly attributed to the dysfunction of synaptic transmission and changes in their physiological characteristics and the surrounding of neurons. Astrocytes are involved in regulating the activity of neurons, the release of transmitters, and play an important role in mental diseases, including schizophrenia, epilepsy, Alzheimer's disease, and depression (Hamilton et al., Frontiers in neuroenergetics 2, 2010). The postmortem brain studies found that the density, morphology and function of glial cell in frontal limbic system significantly changed in major depressive disorder compared with controls (Cotter et al., Archives of general psychiatry 58, 545-553, 2001; Coyle et al., Archives of general psychiatry 57, 90-93, 2000; Rajkowska et al., CNS & neurological disorders drug targets 6, 219-233, 2007). Astrocyte apoptosis by chemical drugs is sufficient to cause depressive symptoms (Banasr et al., Biological psychiatry 64, 863-870, 2008). Antidepressant drugs can act directly on astrocytes, significantly affecting their morphology and function, which have been proposed as one of the antidepressant mechanisms (Czeh et al., the journal of the European College of Neuropsychopharmacology 23, 171-185, 2013). These evidence suggests that in addition to neurons, glial cells also play an important role in mental disease.

The inward rectifier-type potassium channel (Kir) is a hyperpolarization-activated potassium channel, which includes 7 families, Kirl~Kir7. The same kind of Kir channel can be divided into a variety of subtypes due to the existence of splicing variance. The Kir channel distributes in heart, kidney, nervous system and other tissues and organs. Kir4.1 ( also known as potassium voltage-gated channel subfamily J member 10, Kcnj10) is an astrocyte expressed inwardly rectifying potassium channel, mainly responsible for setting the astrocytic RMPs and buffering the excess extracellular K+ in synaptic microdomains. Dysfunction of Kir4.1 will greatly affect the function of glial cells and neurons and contribute to many diseases. In mammals, both the Kir4.1 protein sequence and the coding nucleic acid sequence are conservative. Certain antidepressants have been shown to be inhibitors of Kir4.1 (Ohno et al,. Brain Reseach, 1178, p. 44-51, 2007 and Su et al., Journal of Pharmacology and Experimental Therapeutics, 320(2), p. 573-580, 2007).

There are already some commonly used antidepressants in the field, but these drugs usually take effect after a long period of time. Moreover, the pathological mechanism leading to depression has not been fully recognized. There is a need in the art for new drugs for treating depression, which have a faster onset rate or a safer effective dosage.

### SUMMARY OF THE INVENTION

The inventors of the present disclosure have for the first time and unexpectedly found that Kir4.1 present in astrocytes of the lateral habenula is a crucial regulatory factor of depression. With the assistance of molecular, behavioral and electrophysiological methodologies, the inventors found out that Kir4.1 expressed in astrocytes of the lateral habenula is presented in a form that closely surrounds the cells of habenula neurons. Kir4.1 regulates the extracellular potassium balance and affects the discharging characteristics of the lateral habenula neurons, which leads to over-hyperactivity of the lateral habenula, in turn affecting the phenotype of depression. The inventors of the present disclosure have also discovered and demonstrated various pharmaceutical agents that block the function of Kir4.1 in the lateral habenula, on top of which have thus provided a medicament for treating (suppressing) depression by inhibiting the activity of Kir4.1.

The invention is as defined in the appended set of claims.

In particular, the present disclosure provides a method for treating depression in a subject by inhibiting the activity of Kir4.1. The present disclosure also provides use of a Kir4.1 inhibitor for manufacturing a medicament for treating depression.

Subject to whom the method is applied and the pharmaceutical composition is administered as described in the present disclosure can be a mammal, including a human or a non-human primate such as a monkey. The mammal can be other animals such as rats, mice, rabbits, pigs, dogs, and the like. The mammal can be a domestic animal such as a cat or a dog.

A Kir4.1 inhibitor (or, "an agent inhibiting Kir4.1") is an agent which can decrease or eliminate Kir4.1 channel activity. Kir4.1 channel activity is referred to as a functionality that allows potassium ions to pass through cell membrane. Kir4.1 regulates the potassium ion concentration of the external fluid around the nerve cells and transports excess extracellular potassium ions to buffer the extracellular environment and control the resting membrane potential level, thereby affecting the physiological activity of the nervous system. A Kir4.1 inhibitor includes a compound, a complex, or a mixture that can decrease or eliminate the Kir4.1 channel activity, and may optionally include a reagent that is used in the method for inhibiting Kir4.1 activity (including a surgical method), and the like.

In the method for treating depression by inhibiting the activity of Kir4.1 and the use of a Kir4.1 inhibitor for manufacturing a medicament for treating depression, the Kir4.1 inhibitor is an agent which can affecting the protein level of Kir4.1 so as to affect the Kir4.1channel activity. The agent can be a small molecule compound or complex, or a macromolecular active component such as a protein or a nucleic acid, for example, an antagonist such as an antibody that binds to Kir4.1, or a nucleic acid that affects the expression level of such proteins. These proteins or nucleic acids can be delivered to a target tissue or cell by techniques well known in the art, for example, in conjunction with a suitable expression vector.

In another aspect of the disclosure, the Kir4.1 inhibitor is a selective Kir4.1 inhibitor. A selective Kir4.1 inhibitor is generally referred to as Kir4.1 inhibitor that has no inhibitory activity against other Kir proteins, or has a significantly less inhibitory activity against other Kir proteins in compassion with that against Kir4.1, for example the inhibitory activity against other Kir proteins is 50% or less, preferably 20% or less, more preferably 5% or less, than the inhibitory activity against Kir4.1.

In one aspect of the present disclosure, the Kir4.1 inhibitor is a small interfering RNA or a precursor thereof which suppresses expression of Kir4.1. RNA interference (RNAi) induces efficient and specific degradation of homologous mRNA with double-stranded RNA (dsRNA), thereby reducing or even eliminating the expression of the target gene. In the present disclosure, the interfering RNA may include small interfering RNA (siRNA), short hairpin RNA (shRNA), and/or microRNA (miRNA). One way to administer interfering RNA in vivo is to administer a shRNA (siRNA precursor), such as a short hairpin RNA comprising two short inverted repeats. The siRNA sequence was cloned into a plasmid vector as a "short hairpin". When introduced into cells, the hairpin sequence is expressed to form a "double-stranded RNA", and the corresponding siRNA is generated by the intracellular Dicer enzyme to exert RNA interference.

In yet another aspect of the present disclosure, the small interfering RNA or a precursor thereof used in the present disclosure has identical or complementary sequence with the target mRNA of Kir 4.1, or have at least 90% homology with the identical or complementary sequence. For example, in one aspect of the present disclosure, said small interfering RNA or a precursor thereof which suppresses expression of Kir4.1 has one of the following sequence:
5'-GGACGACCTTCATTGACAT-3' (SEQ ID No. 1);
5'-GCTACAAGCTTCTGCTCTTCT-3' (SEQ ID No. 2);
5'-GCTCTTCTCGCCAACCTTTAC-3' (SEQ ID No. 3);
5'-CCGGAACCTTCCTTGCAAA-3' (SEQ ID No. 4);
5'-GCGTAAGAGTCTCCTCATTGG-3' (SEQ ID No. 5); or
5'-GCCCTTAGTGTGCGCATTA-3' (SEQ ID No. 6).

The above interfering RNA or its precursor targets the sequences of the corresponding mRNA fragments of the rat Kir4.1, which has a sequence of SEQ ID No. 7 (i.e., the CDS region of Genebank No. NM_031602.2). In other words, the sequence of the interfering RNA having one of the sequences of SEQ ID No. 1-6 are identical or complementary to the sequences of the mRNA fragments of rat Kir4.1, or have a homology of more than 90% identical thereto or complementary therewith. The skilled in the field will understand and be able to obtain fragments of the interfering RNA sequence of the Kir4.1 correspondingly in other mammals (e.g., human, mouse, and the like).

In one aspect of the present disclosure, the Kir4.1 inhibitor is a mutant Kir4.1 protein with diminished or deactivated potassium channel activity, or an nucleotide sequence encoding said mutant Kir4.1 protein. A mutant protein can compete with the wide-type protein, thereby reducing the activity or the functionality of the wide-type protein. The mutant protein can be expressed in a target tissue or cell by administering a vector expressible in a target tissue or cell (i.e., the vector carries an expressible mutant protein gene and/or an expression factor thereof). In yet another aspect of the disclosure, the mutant Kir4.1 protein is a mutant Kir4.1 protein with one or more mutations in the channel pore region of Kir4.1. Preferably, said mutant Kir4.1 protein has a mutation at GYG of position 130-132 of the amino acid sequence of wild-type Kir4.1 protein as set forth in SEQ ID NO. 8, for example, said GYG can be mutated to be AAA. Kir4.1 having the amino acid sequence of SEQ ID No. 8 is Kir4.1 (NP_113790.2) of rat. Those skilled in the art can, based on known reports (e.g., Hiroshi et al., 2010. Physiological Reviews 90, 291-366, 2010) and understanding on the conservativeness of the sequence of Kir4.1, obtain the information for channel pore region of Kir4.1 of the other mammals and introduce mutations to Kir4.1 in the channel pore region thereof.

In one aspect of the present disclosure, the Kir4.1 inhibitor is a specific antibody against Kir4.1, including a polyclonal antibody or a monoclonal antibody.

Certain compounds are known in the art for use in the treatment of depression, for example, buspirone, mianserin, fluoxetine, sertraline, fluvoxamine or nortriptyline. However, in these reports, the anti-depression mechanisms that have been discovered or presumed are completely different from the mechanism discovered by the present disclosure, that is, Kir4.1 present in astrocytes of the lateral habenula play an important role in the treatment of depression by inhibiting the abnormal firings, particularly the abnormal burst firing of lateral habenula neurons. Without affecting the novelty and inventive steps of the present disclosure, in one aspect of the present disclosure, in the method for treating depression by inhibiting Kir4.1, the use of Kir4.1 inhibitor in manufacturing a medicament for treating depression, said Kir4.1 inhibitor does not include buspirone, mianserin, fluoxetine, sertraline, fluvoxamine or nortriptyline, and the like. Without affecting the novelty and inventive steps of the present disclosure, in one aspect of the present disclosure, in the method for treating depression by inhibiting Kir4.1, the use of Kir4.1 inhibitor in manufacturing a medicament for treating depression, said Kir4.1 inhibitor is not a selective serotonin reuptake inhibitor (SSRI) or a tricyclic antidepressant (TCA).

In the present disclosure, the term depression may be especially referred to as lateral-habenula-mediated depression, and in more particular may be referred to lateral-habenula-burst-mediated depression. The inventors of the present application have found and demonstrated that the abnormal firing of neurons in the lateral habenula, especially the abnormal burst firing plays an important role in the generation of depression. The inventors of the present application have found and demonstrated that Kir4.1 is a crucial regulatory factor on burst firing, thus a crucial regulatory factor of depression, the inventors of the present application have also identified and proved different types of Kir4.1 inhibitors. The inventors of the present application thereby provide methods and medicaments for treating depressions. This is a pathological mechanism containing the target tissue in the brain and the molecular targets that currently known mechanisms and drugs have failed to target. Accordingly, the methods and medicaments or pharmaceutical compositions provided by the present disclosure are particularly suitable for use in the depression patients to whom the above-described anti-depression methods and drug are ineffective.

In one aspect of the present disclosure, the method and medicaments for treating depression are configured for use in lateral habenula topically (i.e. configured to be locally administered to the lateral habenula). For methods and medicaments/agents used on nerve tissues, particularly the nerve tissues in the brain, such as on the lateral habenula, it is beneficial to limit the effects of the drug/medicament/pharmaceutical agent to the target tissue. The administration of a medicine topically in the lateral habenula is a limiting technical feature to a method and a pharmaceutical agent for treating depression. In any method or pharmceutical agent or medicament for LHb, whether the method or drug can take effects in LHb shall be considered, including whether the drug can reach LHb, and whether the effective concentration can be achieved in LHb, etc. According to some embodiments of the present disclosure, the medicament or pharmaceutical composition is a dosage form for topical administration to the lateral habenula. The action of the medicament can be limited to the target tissue by topical administration, for example by formulating the medicament as a dosage form that can be administered topically to the lateral habenula by cannulation. For another example, the drug is formulated as a dosage form which sustained releases after being implanted into the tissue. The above medicaments can also be formulated in the form of tissue-specific targeted drug delivery systems. For example, a small molecule compound or a biologically active molecule (nucleic acid such as a protein-encoding DNA or mRNA molecule, or a protein such as an antibody, etc.) capable of specifically binding to a protein expressed in the lateral habenula can be used to link with an antibody or fragments thereof which binds to cells of the lateral habenula to form a complex molecule capable of recognizing and binding to lateral habenula.

In one aspect of the present disclosure, in the method for treating depression by inhibiting Kir4.1 in the lateral habenula and the use of a Kir4.1 inhibitor in a medicaments for treating depression which is tropically administered in the lateral habenula, said Kir4.1 inhibitor can include a selective serotonin reuptake inhibitors (such as buspirone, mianserin, fluoxetine, sertraline, fluvoxamine) or tricyclic antidepressants (such as nortriptyline).

The present disclosure also provides a pharmaceutical composition for treating depression, comprising a therapeutically effective amount of Kir4.1 inhibitor, wherein said Kir4.1 inhibitor is as defined and described above.

In one aspect of the present disclosure, in the pharmaceutical composition for treating depression, the Kir4.1 inhibitor is a small interfering RNA or a precursor thereof which suppresses expression of Kir4.1. In one aspect of the present disclosure, the small interfering RNA or a precursor thereof used in the present disclosure has identical or complementary sequence with the target mRNA of Kir 4.1, or have at least 90% homology with the identical or complementary sequence. For example, in one aspect of the present disclosure, said small interfering RNA or a precursor thereof which is capable of suppressing expression of Kir4.1 has the one of the following sequences:
5'-GGACGACCTTCATTGACAT-3' (SEQ ID No. 1);
5'-GCTACAAGCTTCTGCTCTTCT-3' (SEQ ID No. 2);
5'-GCTCTTCTCGCCAACCTTTAC-3' (SEQ ID No. 3);
5'-CCGGAACCTTCCTTGCAAA-3' (SEQ ID No. 4);
5'-GCGTAAGAGTCTCCTCATTGG-3' (SEQ ID No. 5); or
5'-GCCCTTAGTGTGCGCATTA-3' (SEQ ID No. 6).

In one aspect of the present disclosure, in the pharmaceutical composition for treating depression, the Kir4.1 inhibitor is a mutant Kir4.1 protein with diminished or deactivated potassium channel activity, or an encoding nucleotide sequence thereof. Preferably, the mutant Kir4.1 protein is a mutant Kir4.1 protein with a mutation in the channel pore region of Kir4.1. More preferably, said mutant Kir4.1 protein has one or more mutations in GYG of position 130-132 of the amino acid sequence as set forth in SEQ ID NO. 8. For example, said GYG can be mutated to be AAA.

In one aspect of the present disclosure, in the pharmaceutical composition for treating depression, the Kir4.1 inhibitor is a specific antibody against Kir4.1, including a polyclonal antibody or a monoclonal antibody.

Without affecting the novelty and inventive steps of the present disclosure, in one aspect of the present disclosure, in the pharmaceutical composition for treating depression, said Kir4.1 inhibitor does not include buspirone, mianserin, fluoxetine, sertraline, fluvoxamine or nortriptyline, and the like. Without affecting the novelty and inventive steps of the present disclosure, in one aspect of the present disclosure, in the pharmaceutical composition for treating depression, said Kir4.1 inhibitor is not a selective serotonin reuptake inhibitors (SSRIs) or tricyclic antidepressants (TCAs).

The pharmaceutical composition for treating depression provided by the present disclosure are particularly suitable for treating depression patients to whom the other anti-depression methods and drug are ineffective.

In one aspect of the present disclosure, the pharmaceutical composition for treating depression provided by the present disclosure are for use in lateral habenula locally. For example, said pharmaceutical composition is a formulation for use in lateral habenula topically. For these topical pharmaceutical composition for treating depression provided by the present disclosure, said Kir4.1 inhibitor can include a selective serotonin reuptake inhibitors (such as buspirone, mianserin, fluoxetine, sertraline, fluvoxamine) or tricyclic antidepressants (such as nortriptyline).

The disclosure also provides an animal model of depression, preferably which is a rat or a mouse. The animal model of depression according to the present disclosure shows symptoms of depression, which has over-expressed Kir4.1 in the lateral habenula.

The present disclosure also provides a method for screening potential substances for treating depression, comprising the steps of:
(1) in the testing group, adding a test substance to be screened to an *in vitro* testing system;
(2) checking an expression level and/or an activity of Kir4.1 in said *in vitro* testing system in the testing group; comparing it with the control group.

Wherein, if the symptoms associated with depression in the animal model of depression are significantly improved, it indicates that the test substance is a potential substance that can be used to treat depression.

In yet another aspect of the present disclosure, said method further comprises one or more if the following steps:
further testing the effect of the test substance on the burst in neuron; and/or
administering said test substance to an animal model of depression and observing how it affects the symptoms associated with depression.

Wherein, in comparison with the negative control group, if the expression level of Kir4.1 is significantly decreased, and/or the Kir4.1 channel activity is significantly decreased, it indicates that the test substance is a potential substance that can be used to treat depression.

In still another aspect of the disclosure, the method of screening for a potential substance for treating depression further comprises one or more of the following steps:
the potential substances screened in the previous step are further tested for their effects on the burst in neurons; and/or
the potential substances screened in the previous step are administered to animal models to observe their effects on the symptoms of depression.

When testing the effect on the burst in neurons, if the ratio of burst in neurons in the test group to which the test substance was added or administered is significantly lower than that of the negative control group (or the blank control group), it means that test substances are potential substances for the treatment of depression.

### Terminology

'Kir4.1', or 'inward rectifier potassium channel Kir4.1', is also known as potassium voltage-gated channel subfamily J member 10(Kcnj10). Kir4.1 is a member of inward rectifier potassium channel. Kir4.1 in neurogliocytes allows potassium to pass through cell membrane. Kir4.1 regulates the potassium ion concentration of the external fluid around the nerve cells and transports excess extracellular potassium ions to buffer the extracellular environment and control the resting membrane potential level, thus affects the physiological activity of the nervous system. In mammals, the amino acid sequence of Kir 4.1 and the nucleotide sequence encoding it are both very conservative. Gene encoding human Kir4.1 (NP_002232) is KCNJ10(Ensembl: ENSG00000177807). Gene encoding rat Kir4.1 (NP_113790) is *KCNJ10* (Ensembl: ENSMUSG00000044708).

As used herein, "treatment" comprises any of: an ongoing process or outcome that ameliorates, palliates, decreases or prevents the symptoms associated with a medical condition or infirmity; an ongoing process or outcome that improves the symptoms associated with a medical condition or infirmity; an ongoing process or outcome to normalize body functions in disease or disorders that result in impairment of specific bodily functions; or an ongoing process or outcome that elicits an improvement in one or more of the clinically measured parameters of the disease. In one embodiment, a treatment objective is to prevent or slow down (lessen) an undesired physiological condition, disorder or disease, or to obtain beneficial or desired result. The result can be, e.g., medical, physiological, clinical, physical therapy, occupational therapy, subjective to a health care worker or to a patient; or a parameter understood in the art as a "quality of life" or an "activity of daily living". For the purposes of this disclosure, beneficial or desired clinical results comprise, but are not limited to, alleviation of symptoms; diminution/diminishment of the extent of the condition, disorder or disease; stabilization (i.e., not worsening) of the state of the condition, disorder or disease; delay in onset or slowing of the progression of the condition, disorder or disease; amelioration or palliation of the condition, disorder or disease; and remission (whether partial or total), whether detectable or undetectable; or enhancement or improvement of the condition, disorder or disease. In one embodiment, treatment includes eliciting a clinically significant response without excessive levels of side effects. In one embodiment, treatment also includes prolonging survival as compared to expected survival if not receiving treatment. In one embodiment, treatment refers to the administration of medicine or the performance of medical procedures with respect to a patient. As used herein, treatment can be to prophylax (prevention), to cure the infirmity or malady, or to ameliorate the clinical condition of the patient, including a decreased duration of illness or severity of illness, or subjective improvement in the quality of life of the patient or a prolonged survival of the patient.

The term 'burst', or 'burst firing', means a firing pattern in neurons with two or more spikes of plateau potentials (short as spikes hereafter) each time of firing.

The term "inhibiting burst firing", "inhibiting burst", "inhibition of burst" means inhibiting the level of neuronal burst firing, including decreasing of burst frequency or reducing a number of intra-bursting spikes, reducing bursting amplitude and even eliminating burst firing.

The term 'tonic pulse' or 'tonic firing' means a neuronal firing pattern with only one spike in each firing.

In one aspect, the present disclosure also provides a method of diagnosing depression, comprising detecting the expression of Kir4.1 in the lateral habenula of the patient. In one aspect of the disclosure, the method of diagnosing depression is to detect an increase in the expression of Kir4.1 in the lateral habenula of the patient. In yet another aspect of the disclosure, the method of diagnosing depression comprises comparing the expression of Kir4.1 in lateral habenula of the patient at different timings (for example, at different stages of depression, or before or after treatment), or comparing the expression of Kir4.1 in the lateral habenular of a normal subject to that in a general or a specific population. If the expression of Kir4.1 is significantly increased, the subject is diagnoses to have depression.

In one aspect of the disclosure, expression of Kir4.1 can be detected by any methods known in the art for detecting protein (expression) in a sample. The methods can identify the presence or absence of Kir4.1. The methods can also quantitatively detect the amount of expression of Kir4.1.

Methods for detecting a protein (expression) in a sample which can be used in the present disclosure include immunoassay. For example, ELISA or Western blotting carried out with an antibody that specifically recognizes Kir4.1, in which the antibodies can be monoclonal or polyclonal antibodies.

Methods for detecting a protein (expression) in a sample can be used in the present disclosure further comprises detecting the presence or amount of mRNA of Kir4.1, for example, detecting the amount of mRNA of Kir4.1 or a fragment thereof in the sample by RT-PCR.

In one aspect of the disclosure, the test sample is from the lateral habenular of the subject. In one aspect of the disclosure, the test sample is from an ex vivo sample. In one aspect of the disclosure, detection of the expression of Kir4.1 can also be performed by in vivo observation and detection of the amount of Kir4.1 of the lateral habenular of the subject. For example, the test include imaging the lateral habenular of the patient, for example PET imaging. The positron emission tomography (PET) scan of the lateral habenular is carried out by intravenous injection of positron emission radionuclide tracer which recognizes and displays Kir4.1, and followed by a positron emission tomography (PET) scan.

### BRIEF DESCRIPTION OF THE DRAWINGS

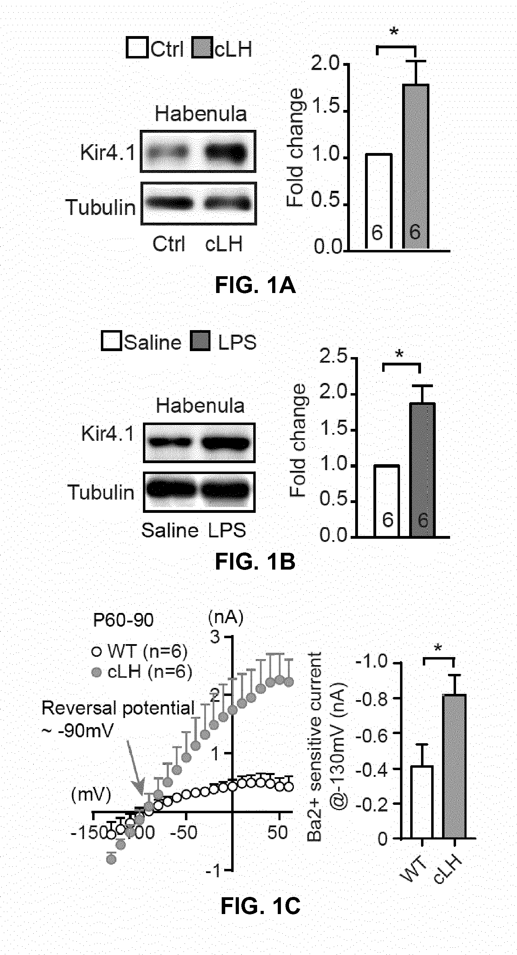

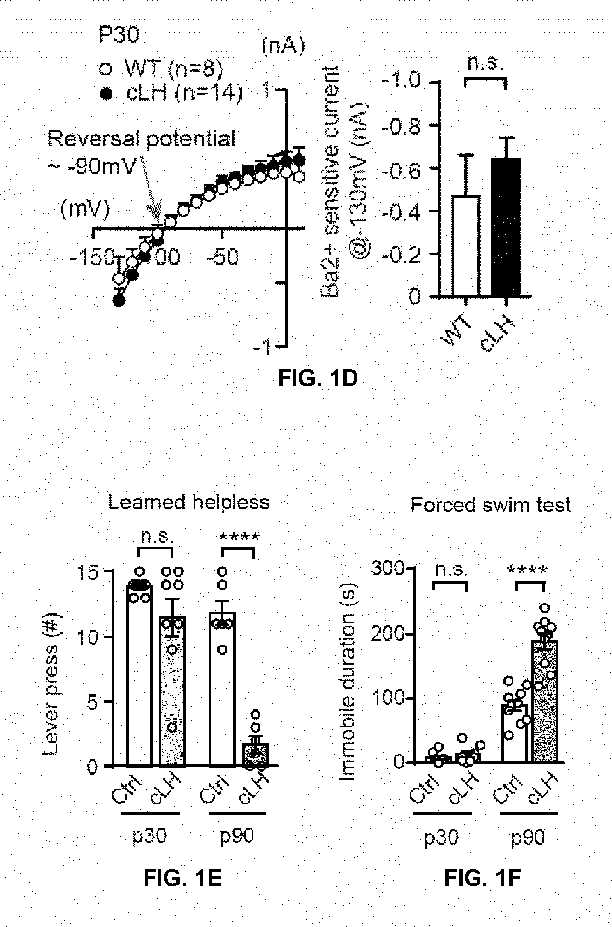

FIG. 1 shows that expression and function of Kir4.1 is upregulated in lateral habenula of rat models of depression. (A, B) Western blot analysis showing upregulation of Kir4.1 in the habenula of cLH (A) and LPS-induced depression rat models (B). Tubulin expressed abundantly in tissues is used as loading control. Protein expression is quantitatively normalized. (C) qPCR analysis of Kir4.1 mRNA in lateral habenula. (D, E) In adult cLH rats (60-90 days of age), but not in juvenile rats (30 days of age), Ba+ sensitive currents (generally mediated by Kir4.1 channels) in the lateral habenula were significantly increased. (F, G) Adult rats, not juvenile cLH rats, exhibited a significant depressive phenotype in the learned helplessness and forced swimming tests. Data are means ± SEM. Numbers in the bars indicate the number of animals used. *P < 0.05, **P <0.01, ***P < 0.001, ****P < 0.0001; n.s., not significant.

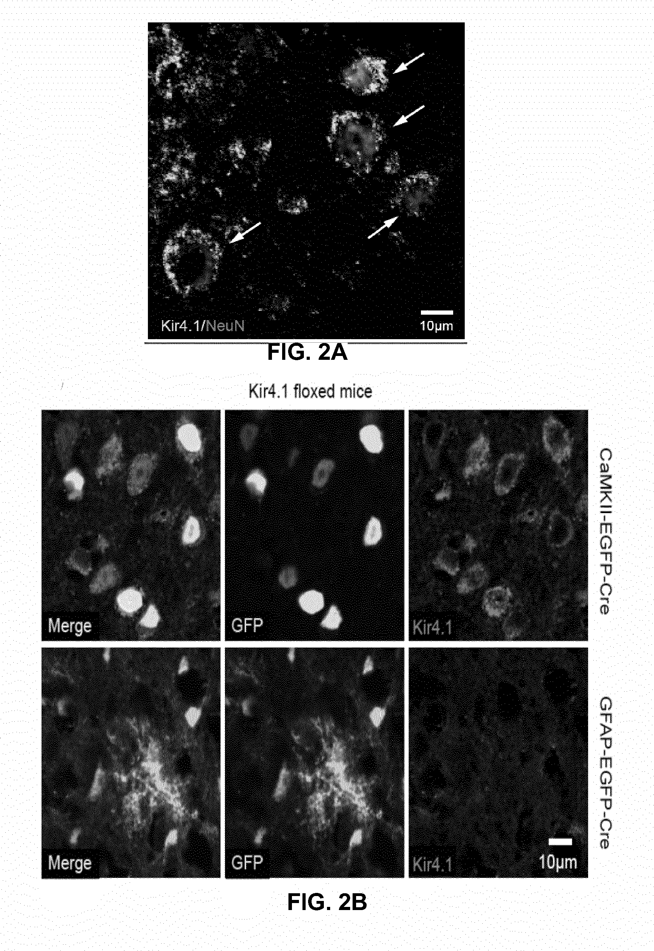

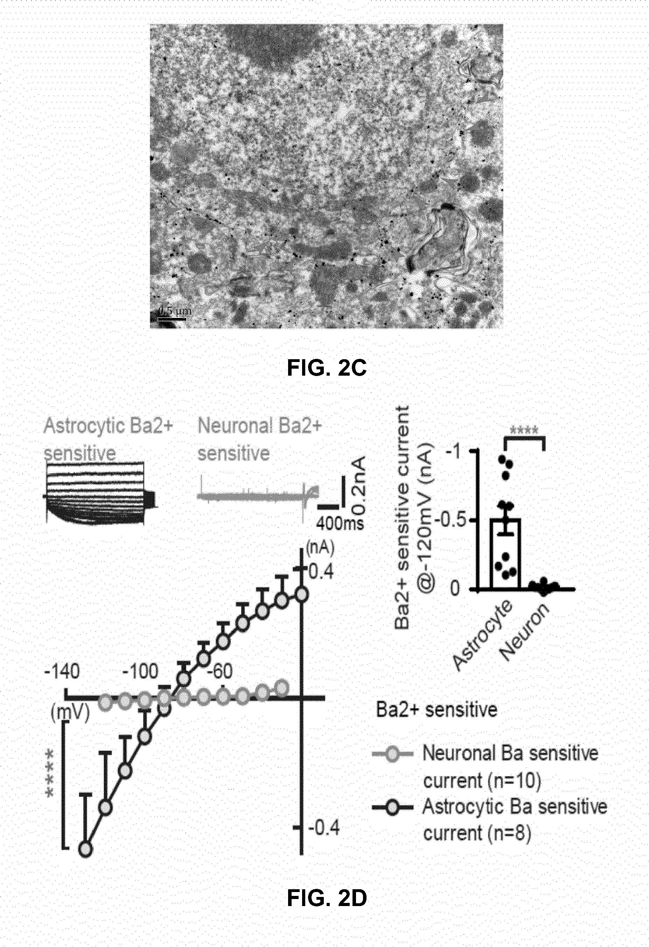

FIG. 2 shows Kir4.1 is expressed on astrocytic processes tightly wrapping around neuronal soma. (A) Immunohistochemistry signals of Kir4.1 wrap neuronal soma as indicated by white arrows. (B) after knocking out Kir4.1 in the neurons of the lateral habenula, the phenomenon of Kir4.1 surrounding the neuron soma still remained; but after knocking out Kir4.1 in astrocytes by injecting with AAV2/5-GFAP-EGFP-Cre virus, the phenomenon of Kir4.1 surrounding the neuron soma disappeared.(C) Immunogold electron microscopy indicate gold signals of Kir4.1 surrounding a neuronal soma. (D) Whole-cell patch-clamp electrophysiological techniques recorded Ba²⁺-sensitive Kir4.1 currents on astrocytes rather than neurons.. Data are means ± SEM. ****P < 0.0001; n.s., not significant.

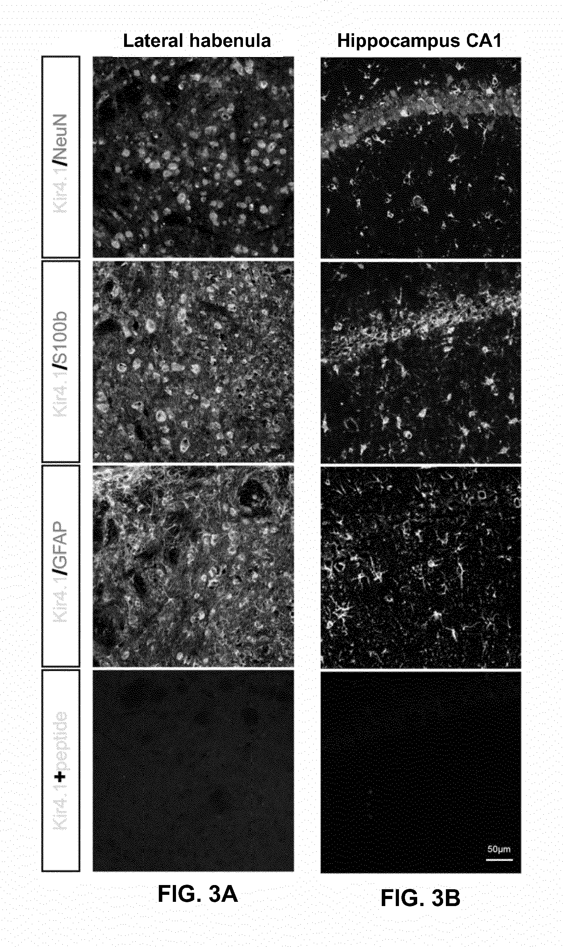

FIG. 3 shows shows the cellular localization of Kir4.1 in the lateral habenula and hippocampus. (A) Kir4.1 co-immunostaining with neuronal marker (NeuN) or astrocytic marker (S100b and GFAP) in LHb. (B) Kir4.1 co-immunostaining with neuronal marker (NeuN) or astrocytic marker (S100b and GFAP) in hippocampus. Bottom two panels are staining with the same kir4.1 antibody pre-incubated with the antigen peptide, demonstrating Kir4.1 antibody.

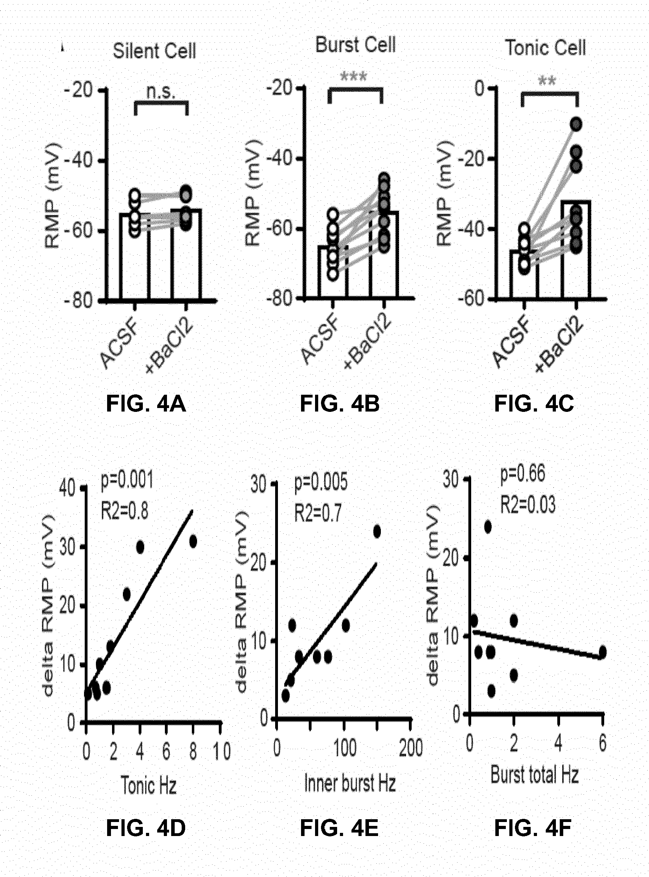

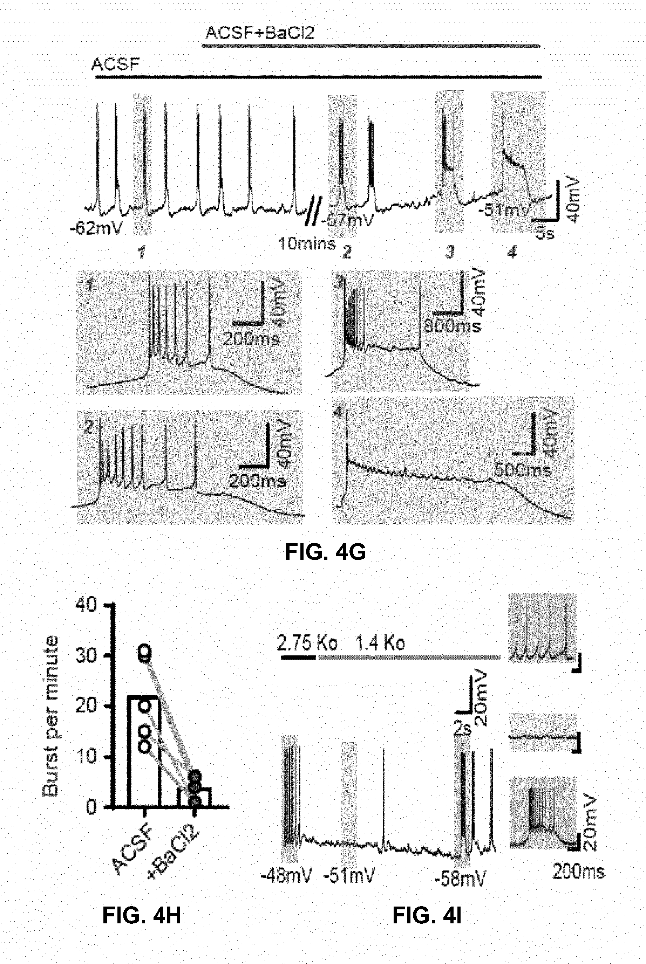

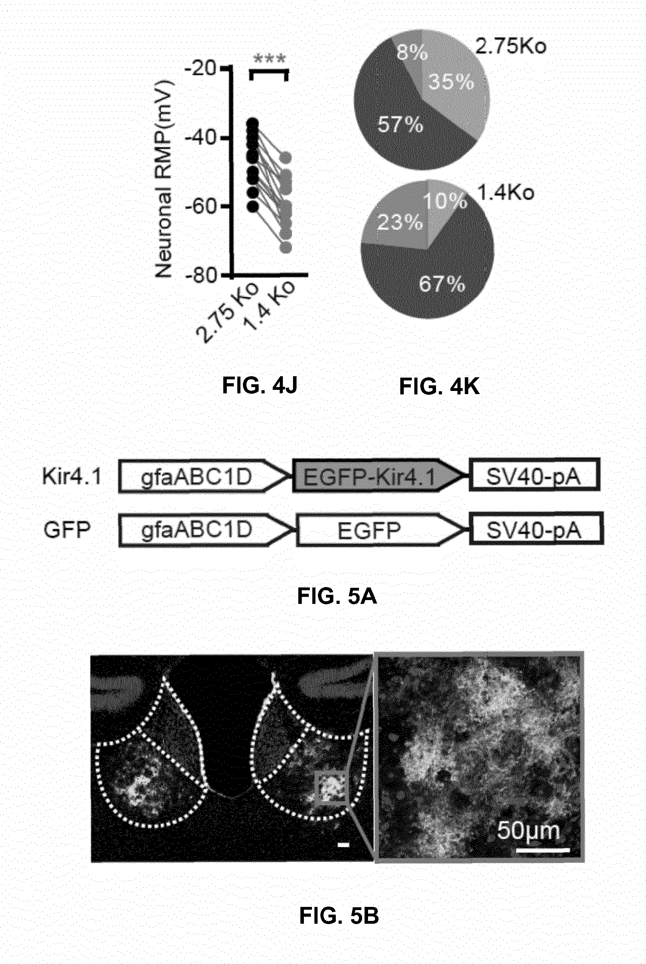

FIG. 4 shows extracelluar potassium is highly correlated with the activity pattern and tightly control by astrocytic kir4.1. (A-C) Changes of neuronal RMPs caused by BaCl₂ (100 µM) in different neuronal types. Extracellular perfusion of artificial cerebrospinal fluid containing BaCl₂ blocks Kir4.1 channel on astrocytes, which can significantly increase the resting membrane potential of burst and tonic neurons in lateral habenula and neurons are shown to be depolarized. (D-F) The degree of neuron depolarization is positively related to its own firing frequency. (G) A typical drawing of the effect of BaCl₂ on neuron firing. (H) BaCl₂ significantly reduced the frequency of burst firing per minute of burst firing cells. (I-K) Decreasing extracellular potassium can significantly reduce the resting membrane potential of neurons and increase the population of burst firing cells. *P < 0.05, **P <0.01, ***P < 0.001, compared with the control group; n.s., not significant.

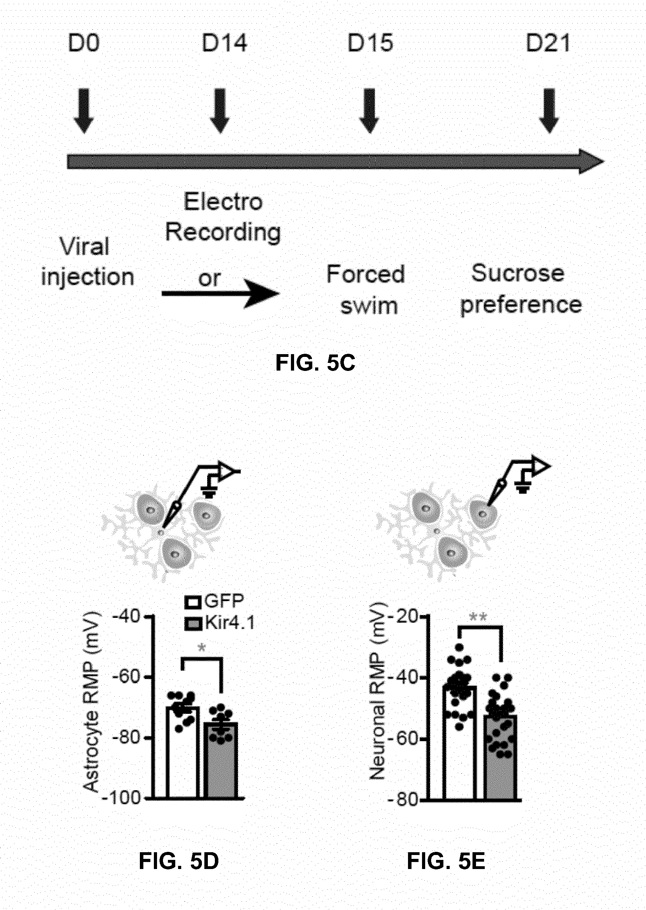

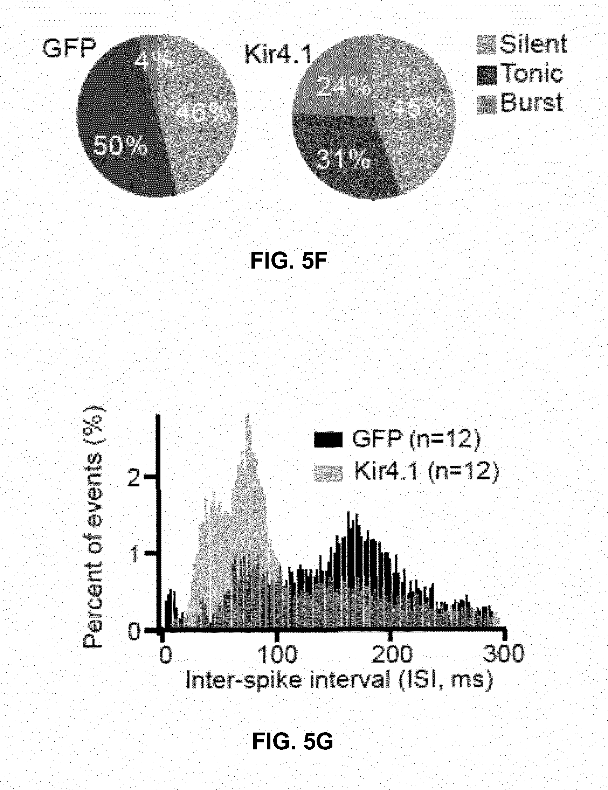

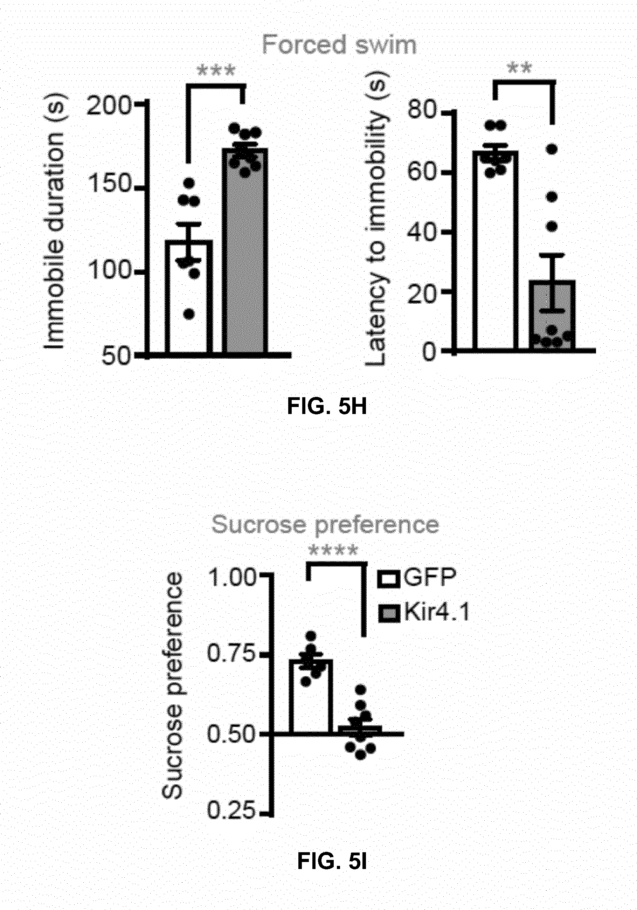

FIG. 5 shows astrocytic kir4.1 overexpression increases population of burst firing cells in LHb and causes depressive-like phenotypes. (A) Schematic diagram of the construction of astrocyte Kir4.1 overexpressed viral vector. (B) Immunofluorescence showed that the virus overexpressed Kir4.1 in astrocytes in the lateral habenula. (C, D) Overexpression of Kir4.1 significantly reduced the resting membrane potentials of both neurons and astrocytes, showing super-polarization. (E) Overexpression of Kir4.1 in astrocytes increases the population of burst firing neurons (F) Overexpression of Kir4.1 in astrocytes increases the population of high-firing-frequency cells (G) Timeline for depression phenotype detection in mice with Kir4.1 overexpressed in astrocytes (H, I) Astrocyte Kir4.1 overexpressed mice showed significant depression phenotypes in both forced swimming and sugar water preference experiments. Data are means ± SEM. **P <0.01, ***P < 0.001, ****P< 0.0001; n.s., not significant.

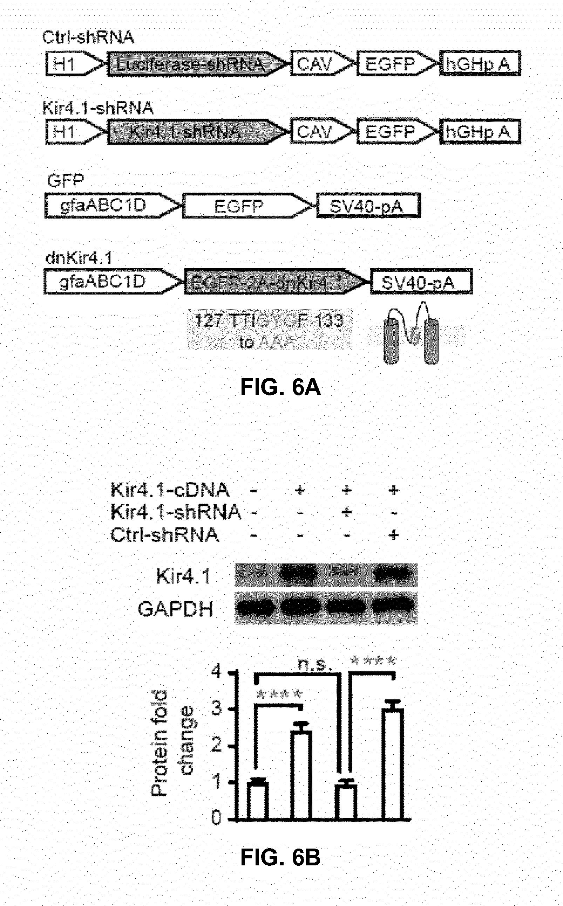

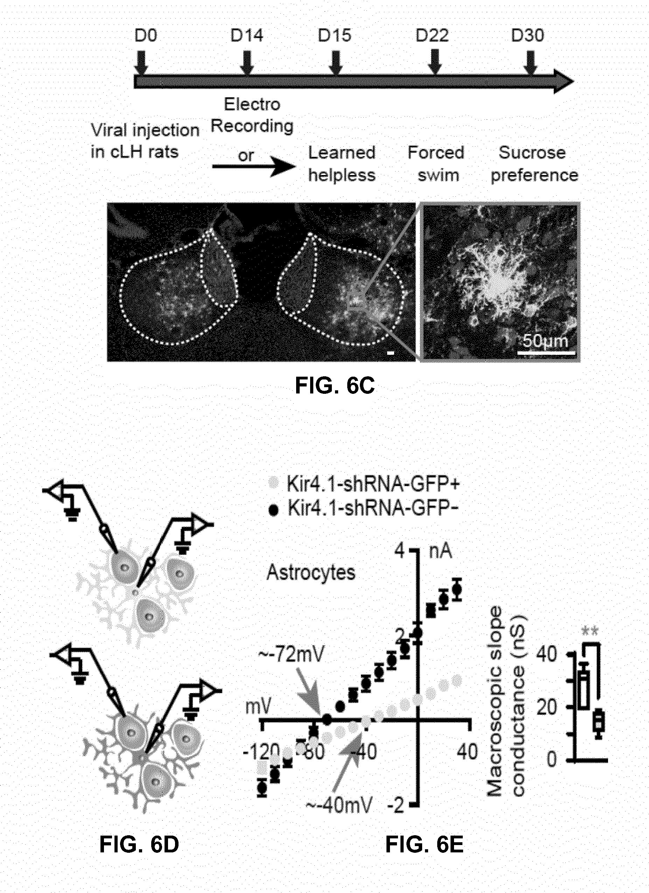

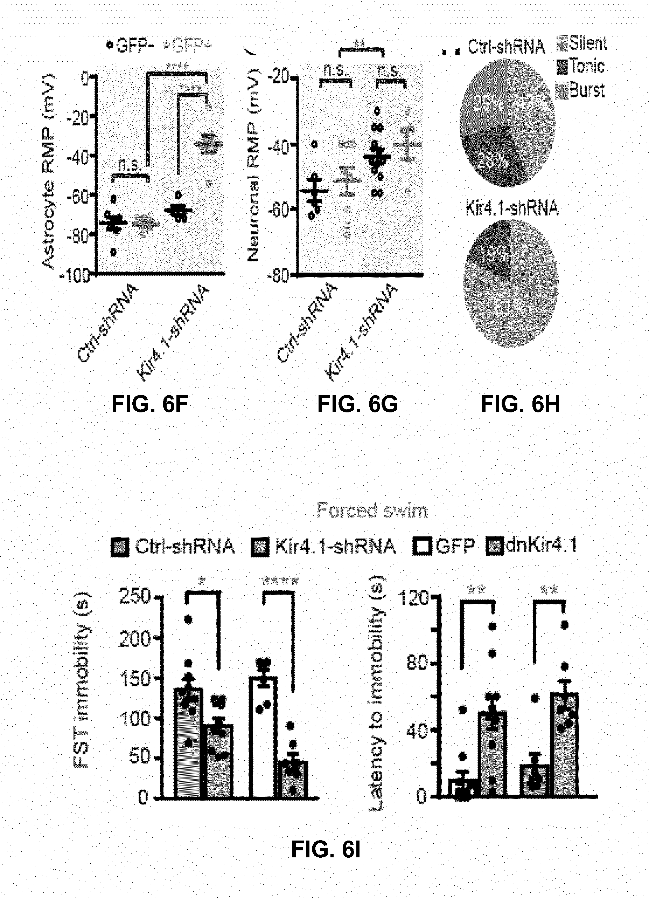

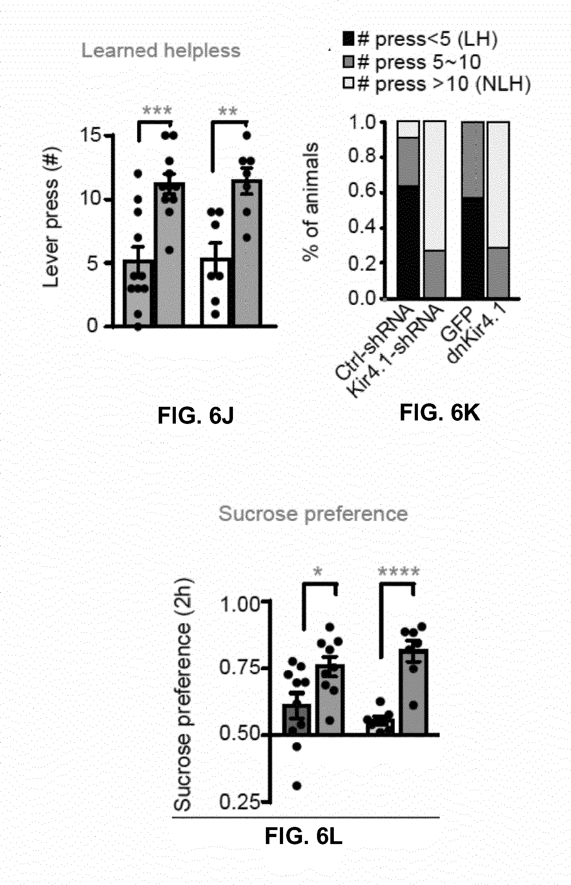

FIG. 6 shows downregulation of function of Kir4.1 in LHb decreases neuronal bursting and rescues depressive-like phenotypes. (A) Schematic diagram of an AAV virus vector used to down-regulate Kir4.1 expression level and a Kir4.1 dominant mutation of Kir4.1 function. (B) Western blot experiments show that in HEK293 cells cultured in vitro, Kir4.1-shRNA can effectively reduce Kir4.1 over-expressed on the cells. (C) The figure on the top shows timeline for electrophysiology experiments and behavioral testing of Kir4.1-function-down-regulated rats. The figure at the bottom shows immunofluorescence of Kir4.1 dominant mutant virus expressed in lateral phrenic astrocytes. (D) The reversal point is higher in astrocytes with down-regulated Kir4.1. (E) The resting membrane potential in astrocytes with down-regulated Kir4.1 was significantly increased, and the resting membrane potential of neurons was also significantly increased, but whether the Kir4.1 were down-regulated in astrocytes does not make difference to the change of membrane potential of neurons. (G) Down-regulation of Kir4.1 expression significantly reduced the population of burst firing cells in the lateral habenula. (H) Down-regulation of Kir4.1 expression and function significantly reduced immobility time in cLH rats during forced swimming. (I, J) Down-regulation of Kir4.1 expression and function rescues depressive phenotype in cLH rats during in Learned helpless test. (K) Down-regulation of Kir4.1 expression and function significantly reversed the loss of sugar-water preference in cLH rats.

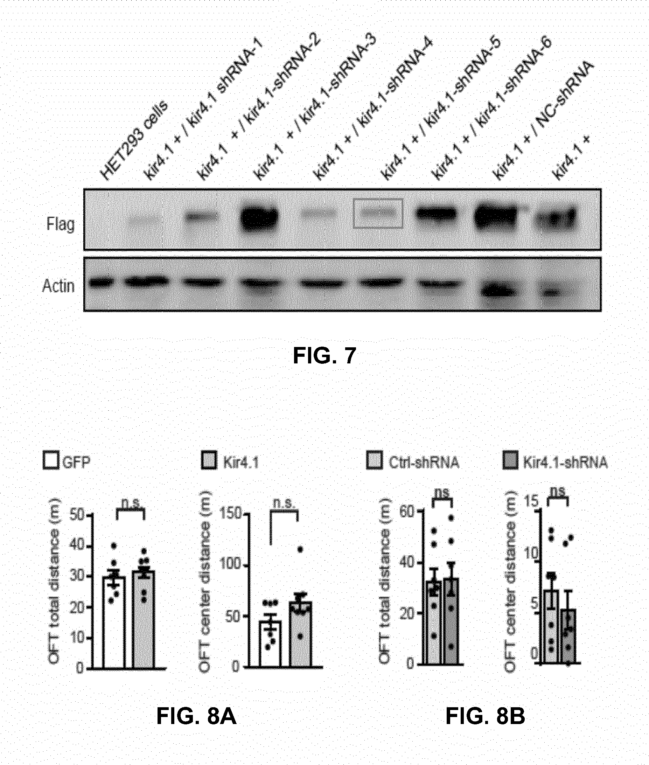

FIG. 7 shows in vitro validation of different Kir4.1 shRNA knockout efficiencies. The Western blotting method was used to detect the exogenous expression of Kir4.1 in HEK293 cells cultured in vitro, and the knockout efficiency of 6 shRNAs targeting different sequences of Kir4.1 was determined.
FIG. 8 shows over-expression of Kir4.1 or down-regulation of Kir4.1 by virus injection in LHb do not affect the animals' mobilization ability. (A) After the expression of AAV2/5-gfaABC1D-EGFP-Kir4.1 virus and overexpression of Kir4.1 in LHb, the total distance of movement and the residence time in the central area of the mice in the open field were not significantly different from those in the GFP control. (B) The expression of AAV2/5-Kir4.1 shRNA virus and the expression of Kir4.1 in LHb were down-regulated. The total distance of motion and central residence time of cLH rats in open field were not different from those of control virus.

### DETAILED DESCRIPTION OF THE PREFERRED EMBODIMENTS

The nature and benefits of the disclosure are further described in the following examples, which are intended to illustrate the disclosure and not to limit the disclosure. The experimental methods without specific descriptions in the following examples are performed according to conventional conditions, such as those described in Sambrook et al., Molecular Cloning: Laboratory Manual (New York: Cold Spring Harbor Laboratory Press, 1989), or according to conditions recommended by the manufacturer.

### Example 1. Materials and methods

Animals. Male cLH rats (4-12 weeks), Sprague Dawley rats (4-12 weeks) and male Wistar rates (12 weeks). The cLH rats is a selective breeding animal model of depression with congenital learned helpless phenotype (D. Schulz, M. M. Mirrione, F. A. Henn, Neurobiol Learn Mem 93, 291, Feb, 2010). The cLH rats used in this study were introduced from the Malinow Laboratory in Cold Spring Harbor, USA. Feeding and propagation of the cLH rat are described in D. Schulz, et al, Feb, 2010 mentioned above. Rats were group-housed four animals/cage under a 12-h light-dark cycle (light on from 7a.m. to 7p.m.). The cLH rats used in cannula infusion experiment were group-housed one animal/cage. Male adult (8-12 weeks of age) C57BL/6 mice were used in behavior tests; mice were group-housed four animals/cage under a 12-h light-dark cycle (light on from 5a.m. to 5p.m.) . Both rats and mice had free access to food and water ad libitum. All animal studies and experimental procedures were approved by the Animal Care and Use Committee of the animal facility at Zhejiang University.

### Virus construction

The Kir4.1 overexpression virus, AAV2/5-gfaABC1D-EGFP-Kir4.1 was prepared, for which a plasmid pZac2.1 gfaABC1D-eGFP-Kir4.1 plasmid (AddGene, Plasmid #52874) was used. The package and preparation of the Kir4.1 overexpression virus was ordered and prepared from Taitool Bioscience of China.

AAV2/1-CamKII-HI-eGFP-Cre, was purchased from University of Pennsylvania Vector core, Upenn, USA , Cat# : AV-1-PV2521.

The Kir4.1 down-regulated virus AAV2/5-H1-Kir4.1-shRNA-gfaABC1D-EGFP (with Kir4.1 interfering RNA-shRNA : 5'-GCGTAAGAGTCTCCTCATTGG-3'), control virus AAV2/5-H1-Luciferase-shRNA-gfaABC1D-EGFP ; Kir4.1 dominant-negative virus AAV2/5-gfaABC1D-Kir4.1dn-2A-EGFP ( GYG of position 130-132 in the channel pore region of Kir4.1 were mutated to be AAA and showed dominant-negative ) were ordered and prepared from Taitool Bioscience of China.

### LPS-induced depression model.

Adult (12 weeks) Wistar male rats were was intraperitoneal injected with LPS at a dosage of 500µg /kg body weight daily for 7 days. The forced swim test was performed 24 hours after the last injection. The habenular tissue was dissected 3 days for expression analysis.

### Stereotaxic surgery and virus injection.

Mice virus injection Mice were anesthetized by intraperitoneal injection of a mixture of ketamine (100 mg/kg body weight) and xylazine (8mg/kg body weight), and fixed on a stereotactic instrument (Stoelting instruments). Each mouse was injected with 0.1-0.2ul purified and concentrated AAV virus (~ 10¹³ infection units / ml) on each side of LHb. LHb localization relative to bregma (AP, -1.72 mm; ML, ±0.46 mm; DV, -2.62 mm from the brain surface). Virus injection was performed using a hand-made mircoinjection needle at a slow rate of 100-150nl/min. After the injection was completed, 5 minutes were allowed to pass and leaving it for an additional 5 minutes before the needle was then slowly withdrawn completely.

Rats virus injection: Rats were anesthetized by intraperitoneal injection of barbital sodium (60mg/kg body weight), and fixed on a stereotactic instrument (Stoelting instruments). Each mouse was injected with 0.1-0.2ul purified and concentrated AAV virus (~ 10¹³ infection units / ml) on each side of LHb. LHb localization relative to bregma (AP, -3.7 mm; ML, ±0.7 mm; DV, -4.55 mm from the brain surface). Virus injection was performed using a hand-made mircoinjection needle at a slow rate of 100-150nl/min. After the injection was completed, 5 minutes were allowed to pass and leaving it for an additional 5 minutes before the needle was then slowly withdrawn completely.

At least 14 days after injection, mice or rats were used in behavioral or electrophysiological studies. All the injection sites will be checked by immunostaing after the behavioral experiments. Only mice with correct injection site will be counted into the behavioral statistics. The GFP-labeled virus was checked for GFP protein with antibodies before microscopy. The habenular nucleus area of each brain was cut into 6 groups of consecutive sections (30 µm sections for mice, 6 sections per group; 40 µm sections for rats, 8-9 sections per group). All sections were counterstained with Hoechst prior to mounting on the mount.

### Immunohistochemistry.

Animals were anesthetized using 4% barbital sodium, and then perfused transcardially with PBS followed by 4% paraformaldehyde. After overnight post fix in 4% paraformaldehyde solution, brains were cryoprotected in 30% sucrose for 1 day to 3 days. Coronal sections (40 µm) were stored at -20°C in protection solution. The antibodies used were anti-Kir4.1(1 :200 , Alomone labs) ,anti-NeuN(1 :500 ,chemicon) ,anti-GFAP(1:500, Chemicon), anti-S100b (1:500,Invitrogen , ABCAM), anti-GFP(1:1000, abCaM), Alexa Fluor488 goat anti-rabbit IgG, Alexa Fluor488 goat anti-chicken IgG, Alexa Fluor594 goat anti-mouse IgG (all 1:1000, Invitrogen), Hoechst ( 1:5000). Fluorescent image acquisition was performed with an Olympus Fluoview FV1000 confocal microscope and a Nikon A1 confocal microscope.

### Western Blot.

Rats were anesthetized with isoflurane and their brains were collected. habenular tissues were separated on ice and then stored freezed in liquid nitrogen. The samples were subjected to tissue disruption by homogenation. The tissue homogenate was centrifuged at 800 g for 15 minutes at 4 degrees. The supernatant was collected and centrifuged at 10000g for 15min. The precipitate was the membrane protein component, which was then dissolved. Samples of the 293TN cell line were lysed by RIPA solution (20 mM Tris-HCl [pH 7.5], 150 mM NaCl, 1 mM EDTA, 1 mM EGTA, 1% NP-40, 1% sodium deoxycholate, 1 mM PMSF, 10 µg / ml aprotinin, 1 µg / ml pepstatin A and 1 µg / ml leupeptin) and centrifuged at 10,000 g for 15 min; the supernatant was collected. After protein concentration measurement by BCA assay, 8~15µg proteins for each lane was separated on a 10% SDS-PAGE gel and transferred for western blot analysis. Anti-Kir4.1 (1:1000, Alomone labs), anti-GFAP (1:1000, Sigma) and anti-tubulin (1:5000, Bio-Rad) antibodies were used. High sensitive ECL reagent was used (GE Healthcare). All the bands were analyzed with Quantity one or Image J.

### Electrophysiological assay.

Rats (P40-50days) and mice (p8 weeks) were anesthetized with isoflurane, and then perfused with 20 ml ice-cold oxygenated ACSF (118 mM NaCl, 2.5 mM KCl, 26 mM NaHCO₃, 1 mM NaH₂PO₄, 10 mM glucose, 1.3 mM MgCl₂ and 2.5 mM CaCl₂, gassed with 95% O₂ and 5% CO₂). The brain was removed as quickly and put into chilled and oxygenated ACSF. Coronal slices containing habenular (350 µm-thickness) were sectioned in cold ACSF. Slices were allowed to recover for at least 1 hour before recording.

For LHb neuron recordings, a MultiClamp 700B amplifier (Axon Instruments) were used currents were measured under whole-cell patch clamp using pipettes with a typical resistance of 4-6 MΩ filled with internal solution containing (mM) 105 K-Gluconate, 30 KCl, 4 Mg-ATP, 0.3 Na-GTP, 0.3 EGTA, 10 HEPES and 10 Na-phosphocreatine, with pH set to 7.35. The external ACSF solution contained (in mM) 125 NaCl, 2.5 KCl, 25 NaHCOs, 1.25 NaH₂PO₄, 1 MgCl₂, 2.5 CaCl₂ and 25 Glucose. Cells were visualized with infrared optics on an upright microscope (BX51WI, Olympus). A MultiClamp 700B amplifier and pCLAMP10 software were used for electrophysiology (Axon Instruments). The series resistance and capacitance was compensated automatically after stable Giga seal were formed. The spontaneous neuronal activity was recorded under current-clamp (I=0 pA) for consecutive 60s. RMP was determined during the silent period of the neuronal spontaneous activity.

To test TTX (1 µM, Sigma) and BaCl₂ (100 µM, Sigma) effect onto neuronal RMP, baselines of RMP were recorded for at least for 3 min. Drug were then perfused, the arriving of the drug was precisely indicated with a bubble that pre-added before the transition from normal ACSF to drug added ACSF. TTX acts on LHb neuron as quickly as several minutes while BaCl₂ takes more 10 min to affect neuronal RMP. The drug effect of TTX and BaCl₂ onto neuronal RMP at the time point of 5 min and 15 min were then tested respectively.

### Astrocytic patch and Kir4.1 current isolation.

Astrocytes were distinguished from neuron by their small (5-10 µm) oval shaped somata and by electrophysiological features: a hyperpolarized RMP and a low input resistance, a linear I-V relationship and an absence of action potentials in response to increased injection currents. BaCl₂ (100 µM, Sigma) were applied to isolate Kir4.1 current which is subtracted from the IV curve recorded from -120 mV to 0 mV.

The patch-clamp recording of LHb neuron slices was recorded with an Axon Multiclamp 700B amplifier at 32 ± 1 °C under an Olympus microscope equipped with an infrared differential interference phase contrast optical lens. The astrocyte recording electrode has an impedance of 7-10 MS2, and the neuron recording electrode has an impedance of 4-6 MS2. All cells were recorded in whole cell mode. Based on the morphology of the recorded cells, the recorded electrophysiological characteristics whether a neuron (15-20 microns in diameter) or an astrocyte (5-10 microns in diameter) was observed. Neuron recording parameters include cell membrane potential, membrane impedance, self-release frequency of action potential (I=0 record), neuron voltage and current curve, and neuron input and output excitability. Astrocyte recording parameters include membrane potential, membrane impedance, and voltage-current curve (by voltage clamp, voltage from -130mV to 40mV, each recording increases by 10 mV, and the current response at each potential was recorded). Kir4.1 current calculation method: the voltage-current curve obtained by adding barium chloride (100 mM, which specifically antagonizes Kir4.1 current) was subtracted from the voltage-current curve recorded in normal artificial cerebrospinal fluid. The data was filtered at 2kHz using a Digidata 1322A, sampling and recording at 10kHz. Data were analyzed using pClamp 10 software.

### Behavior tests

### Learned helpless test (LHT).

The experiments were performed on littermate cLH or wild-type SD rats. It was divided into two sections: the "training section" included 120 unavoidable, uncontrolled 0.8mA plantar electrical stimulation for more than 40 minutes in the stimulation room (Coulbourn Instrument); the random stimulation duration and interval stimulation duration ranging from 5 to 15 seconds, with a total stimulation duration of 20 minutes; the "test section" training was performed 24 hours later, and the learned helpless phenotype was evaluated by a rod compression task during which a light indicating rod was placed in the stimulation room. This part includes 15 evasable 0.8 mA intensity electrical stimuli and 24 s experiment interval. Each stimulus lasted for 60 seconds, but could be terminated by rod compression. Animals which failed to press the lever for more than 10 trials were defined as "learned helplessness" (LH), and rats with less than 5 failures were defined as "non-learned helplessness" (NLH).

### Forced swim test (FST).

The test was performed under normal daylight lamp. Animals were placed in a cylinder (12 cm diameter, 25 cm height). Water depth was 14cm and temperature was 23-24°C. Swimming performance was videotaped from the side for 6 mins. The immobility time during the last 4 min within the 6 min testing period was counted in a double-blind way. Immobility was defined as time when animals remained floating or motionless in the water.

### Sucrose preference test (SPT).

Animals were single housed for a week and habituated with two bottles of water for 2 days, followed by two bottles of 2% sucrose for 2 days. Animals were then exposed to one bottle of 2% sucrose and one bottle of water for 2 h in the testing phase. Bottle positions were switched every 12h. Total consumption of each fluid was measured every 24 hours and for 48 hours in total.

### Statistical analyses.

All the data were given as Means±SEM. All behavior testing data were subjected to two-tailed Student's t-tests or Mann-Whitney test.

### Example 2. Expression and function of Kir4.1 was upregulated in LHb of animal models of depression.

As showed in FIG. 1, expression and function of Kir4.1 was observed in two depression rat models: cLH rats model of depression with congenital learned helpless phenotype, and LPS-induced depression rat model: 3-month Wistar male which were intraperitoneal injected with LPS at a dosage of 500 µg/kg body weight daily for 7 days and then subjected to the forced swim test 24 hours after the last injection.

Western blot analysis results showed in FIGS 1 A and B confirmed that expression of Kir4.1 had a significant increase in lateral habenula of cLH rat and LPS-induced depression rat than that in the control group. FIG. 1A shows expression of Kir4.1 had a 1.75-fold increase in lateral habenula of cLH rat than that in the control group. The Kir4.1 level was also significantly increased (1.87-fold) in lateral habenula of the LPS-depression rats (FIG. 1B).

To determine whether Kir4.1 function was also enhanced in depressive rats, whole-cell patch clamp was performed onto the astrocytes in brain slices made from the LHb of cLH or SD rats. Astrocytes were distinguished from neurons by their small (5-10 µM) oval shaped somata and electrophysiological features including a relatively hyperpolarized RMP (-74 ± 1 mV), a low input resistance Rin (47 ± 6 MS2), a linear I-V relationship and an absence of action potentials in response to depolarizing current injections (FIG. 1C). Ba²⁺ (BaCl₂, 100 µM) was then bath applied, which selectively blocks Kir channels at sub-mM concentrations, to isolate Kir4.1 current. Ba²⁺-sensitive currents in LHb astrocytes were almost doubled in cLH rats, compared with SD controls, at the age of P60-90 (FIG. 1C). Interestingly, the increase of Kir4.1 current was not obvious for non-adult rats (FIG. 1D), at which age, cLH rats did not yet show depressive-like phenotypes in both learned helpless test (LHT, FIG. 1E) and the forced swim test (FST, FIG. 1F), suggesting that level of Kir4.1 overexpression correlated with the developmental onset of the depressive-like symptoms.

### Example 3. Expression pattern of Kir4.1 in the lateral habenula

Surprisingly, the inventors found out for the first time in the field by using immunohistochemistry co-labeling that Kir4.1 were wrapping around neuronal soma in LHb, although in the same brain slice Kir4.1 staining patterns in hippocampus were typical astrocytic-looking (FIG. 3).

FIG. 2 shows that Kir4.1 in LHb was expressed on the astrocyte processes and tightly enclosed the neuronal soma in the lateral habenula.

FIG. 2A shows that the Kir4.1 immunopositive signal surrounded the neuronal soma by immunofluorescence double-labeling staining.

In addition, the expression of Kir4.1 was observed and judged by using a conditional knockout method. Conditional knockout was achieved by injecting adenovirus AAV2/1-CaMKII-EGFP-Cre into the lateral habenula of Kir4.1 conditional knockout mice. As shown in FIG. 2B, after knocking out Kir4.1 in the neurons of the lateral habenula, the phenomenon of Kir4.1 surrounding the neuron soma still remained; but after knocking out Kir4.1 in astrocytes by injecting with AAV2/5-GFAP-EGFP-Cre virus, the phenomenon of Kir4.1 surrounding the neuron soma disappeared.

The immunoelectron microscopy method was also used to detect the distribution of Kir4.1 by showing immune gold particles were around the neuronal cell membrane, but much less at the synaptic sites of the neurons (as shown in FIG. 2C).

Electrophysiological evidence also found that Ba²⁺-sensitive Kir4.1 currents were recorded mainly in astrocytes rather than neurons (as shown in FIG. 2D).

FIG. 3 shows the cellular localization of Kir4.1 in the lateral habenula and hippocampus. FIG. 3A shows the co-localization of Kir4.1 with neuron marker NeuN, astrocyte markers GFAP and S100b in the lateral habenula; FIG. 3B shows the co-localization of Kir4.1 with neuron marker NeuN, astrocyte markers GFAP and S100b in the hippocampal CA1 region. The bottom two figures in FIG. 3 show that after Kir4.1 antibody was bound by Kir4.1 extracellular peptide antigen, no positive signal was detected in the lateral habenula and hippocampal CA1, suggesting that the antibody used was specific to Kir4.1.

The above results indicate that Kir4.1 on astrocyte processes in LHb mainly exists in a form that is tightly wrapped around the neuronal soma.

### Example 4. Regulation of Kir4.1on neuron activity

The effects of Kir4.1 on neuronal activity were studied by patch-clamp electrophysiological recording. Firstly, the artificial cerebrospinal fluid containing BaCl₂ was perfused extracellularly to block the function of Kir4.1 of buffering potassium. It was found that blocking Kir4.1 with BaCl₂ depolarized LHb RMPs of tonic and burst-firing, but not silent neurons. As shown in A-C in FIG. 4, extracellular perfusion of artificial cerebrospinal fluid containing BaCl₂ blocks the Kir4.1 channel on the astrocytes, allowing the resting membrane potential of burst and tonic neurons in lateral habenulato be increased significantly and neurons be depolarized. This is consistent with the increase in extracellular potassium concentration calculated by the Nernst equation, which causes the membrane potential of neurons to be super-high. And the degree of depolarization of LHb self-released cells by BaCl₂ treatment was positively correlated with the frequency of the cells themselves (as shown in D-F in Fig. 4). BaCl₂ treatment for a long time over-excited burst firing neurons, leaving the neurons in a state of rigidity, and ultimately reduced the number of burst firing significantly (figures G-H shown in FIG. 4).

### Example 5. Kir4.1 changed electrophysiological functions of neurons and astrocytes by regulating extracellular potassium concentration

Incubating artificial cerebrospinal fluid with reduced potassium concentration (2.75mM-1.4mM) on LHb brain slices of normal rats, which made the resting membrane potential of neurons to 10.3mV (as shown in Figures I and J of FIG. 4), and percentage of bursting neurons was increased from 8% to 23% (FIG. 4K). In summary, by increasing astrocytic Kir4.1 expression or decreasing the extracellular K+ concentration, it was able to hyperpolarize RMPs and enhanced bursts.

### Example 6. Overexpression of kir4.1 in LHb astrocytes caused depressive syndromes in animal in vivo tests

A Kir4.1 dominant negative mutation virus pAAV-gfaABC1D-Kir4.1dn-2A-EGFP (amino acid residues GYG in the channel pore region of Kir4.1 were mutated to be AAA to achieve a dominant negative mutation in Kir4.1) was construtcted.

### Construction of pAAV-gfaABC1D-Kir4.1dn-2A-EGFP plasmid:

1. Firstly the pAAV-Ubi-Kir4.1-2A-EGFP plasmid was constructed by using pAAV-Ubi-CaMKIIb-2A-EGFP plasmid (Li et al., 2013) as a template, and by using the primers pAAV-ubi Fusion Fw and pAAV-ubi Fusion Rev to amplified a linearized vector; pZac2.1- gfaABC1D-EGFP-Kir4.1 (purchased from AddGene, Plasmid # 52874) was used as a template, and pAAV-ubi-Kir4.1 Fusion Fw and Rev was used to amplify the kir4.1 fragment. The two linearized fragments obtained above were then ligased and yielded pAAV-Ubi-Kir4.1-2A-EGFP.

**Table 1**

| | |
|---|---|
| pAAV-ubi-Kir4.1 Fusion Fw | GCAGGTCGACTCTAGATGACATCAGTTGCCAAGGTCTATTACAGC |
| pAAV-ubi-Kir4.1 Fusion Rev | GCCTACCGGTGGATCGACGTTACTAATGCGCACACTAAGGGC |
| pAAV-ubi Fusion Fw | GATCCACCGGTAGGCAGTGGAGAG |
| pAAV-ubi Fusion Rev | CTAGAGTCGACCTGCAGCCCAAGCT |

2. Construction of pAAV-Ubi-Kir4.1dn-2A-EGFP plasmid: using pAAV-Ubi-Kir4.1-2A-EGFP plasmid as a template, and using the primers Kir4.1 GYG-AAA Fusion Fw and Kir4.1 GYG-AAA Fusion Rev as shown in Table 2 to amplify a linearized mutant fragment, and then ligasing them to be a complete AAA mutant plasmid, namely pAAV-Ubi-Kir4.1dn-2A-EGFP.

**Table 2**

| | |
|---|---|
| Kir4.1 GYG-AAA Fusion Fw | AAAGATGGCCGGAGCAACGTGAGAATGGAGCATATTGCT |
| Kir4.1 GYG-AAA Fusion Rev | GCTGATGTAGCGGAAGGCGGCGGCAATGGTGGTCTGGGATTCGA GGGA |

3. Construction of the pAAV-gfaABC1D-Kir4.1dn-2A-EGFP plasmid: using the pZac2.1-gfaABC1D-EGFP-Kir4.1 plasmid as a template and using the primers pZac2.1 gfaABC1D Fusion Rev and pZac2.1 gfaABC1D Fusion Fw shown in Table 3. to amplify the pZac2.1 gfaABC1D linear fragment lacking of EGFP-Kir4.1; then using the pAAV-Ubi -Kir4.1dn-2A-EGFP plasmid as a template, and using the primers Kir4.1dn-2A-eEGFP Fusion Rev and Kir4.1dn-2A-eEGFP Fusion Fw to amplify the Kir4.1dn-2A-EGFP fragment, then ligasing the above two linearized fragments to obtain pAAV-gfaABC1D-Kir4.1dn-2A-EGFP plasmid.

**Table 3**

| | |
|---|---|
| Kir4.1dn-2A-eEGFP Fusion Rev | CGGGTCGACTCTAGATTACTTGTACAGCTCGTCCATGCCG |
| Kir4.1dn-2A-eEGFP Fusion Fw | TAGGCTAGCGCCACCATGACATCAGTTGCCAAGGTCTATTACA GC |
| pZac2.1 gfaABC1D Fusion Rev | GGTGGCGCTAGCCTATAGTGAGTCG |
| pZac2.1 gfaABC1D Fusion Fw | TCTAGAGTCGACCCGGGCGGCC |

pAAV-gfaABC1D-Kir4.1dn-2A-EGFP was injected into lateral habenula by stereotactic surgery. 14 days after bilateral injection in the LHb, Kir4.1 was over-expressed in astrocytes throughout the LHb. FIG. 5A shows a schematic diagram of the construction of a viral vector for the overexpression of Kir4.1 in astrocytes. FIG. 5B is an immunofluorescence image showing that the virus in the lateral habenula overexpresses Kir4.1 protein in astrocytes. Whole-cell recordings were made from either astrocytes or neurons surrounding the viral-transfected astrocytes in coronal LHb slices. The RMPs of both astrocytes and neurons were more hyperpolarized (FIG. 5D, E) and the percentage of bursting neurons were significantly higher (FIG. 5F, G).

Overexpression of Kir4.1 in the LHb significantly increased immobile duration and decreased latency to immobility in FST and decreased sucrose preference in the sucrose preference test (FIG. 5H, I), while the mobility of mice were not changed (FIG. 8A).

These results showed overexpression of Kir4.1 in the LHb led to typical depressive syndromes.

### Example 7. Downregulation of Kir4.1 expression in the lateral habenula or Kir4.1 function reverses depression phenotype

AAV2/5 virus expressing Kir4.1 short hairpin RNA (shRNA) was used to down-regulate LHb Kir4.1 protein expression in cLH rats.

The six Kir4.1 interfering RNAs constructed according to the table below were cloned into the WX231-L vector (purchased from Taitool Bioscience of China, Cat #: WX231):

| Sticky end | Target Fwd. | Loop | Target Rev | PolyT |
|---|---|---|---|---|
| TCCCC | GGACGACCTTCATT GACAT | TTCAAGAGA | ATGTCAATGAAGG TCGTCC | TTTTT |
| TCTAAAAAA | GGACGACCTTCATT GACAT | TCTCTTGAA | ATGTCAATGAAGG TCGTCC | G |
| TCCCC | GCTACAAGCTTCTG CTCTTct | TTCAAGAGA | AGAAGAGCAGAAG CTTGTAGC | TTTTT |
| TCTAAAAAA | GCTACAAGCTTCTG CTCTTct | TCTCTTGAA | AGAAGAGCAGAAG CTTGTAGC | G |
| TCCCC | GCTCTTCTCGCCAA CCTTTAC | TTCAAGAGA | GTAAAGGTTGGCG AGAAGAGC | TTTTT |
| TCTAAAAAA | GCTCTTCTCGCCAA CCTTTAC | TCTCTTGAA | GTAAAGGTTGGCG AGAAGAGC | G |
| TCCCC | GCCGGAACCTTCCT TGCAAA | TTCAAGAGA | TTTGCAAGGAAGG TTCCGGC | TTTTT |
| TCTAAAAAA | GCCGGAACCTTCCT TGCAAA | TCTCTTGAA | TTTGCAAGGAAGG TTCCGGC | G |
| TCCCC | GCGTAAGAGTCTCC TCATTGG | TTCAAGAGA | CCAATGAGGAGAC TCTTACGC | TTTTT |
| TCTAAAAAA | GCGTAAGAGTCTCC TCATTGG | TCTCTTGAA | CCAATGAGGAGAC TCTTACGC | G |
| TCCCC | GCCCTTAGTGTGCG CATTA | TTCAAGAGA | TAATGCGCACACTA AGGGC | TTTTT |
| TCTAAAAAA | GCCCTTAGTGTGCG CATTA | TCTCTTGAA | TAATGCGCACACTA AGGGC | G |

Sequences of primers used for the cloning is listed in the table below:

**Table 4 primers**

| No. | Primer sequence (5'-3') |
|---|---|
| 1-F | TCCCCGGACGACCTTCATTGACATTTCAAGAGAATGTCAATGAAGGTCGTCCTTTTT |
| 1-R | TCTAAAAAAGGACGACCTTCATTGACATTCTCTTGAAATGTCAATGAAGGTCGTCCG |
| 2-F | TCCCCGCTACAAGCTTCTGCTCTTctTTCAAGAGAAGAAGAGCAGAAGCTTGTAGCTTTTT |
| 2-R | TCTAAAAAAGCTACAAGCTTCTGCTCTTctTCTCTTGAAAGAAGAGCAGAAGCTTGTAGCG |
| 3-F | TCCCCGCTCTTCTCGCCAACCTTTACTTCAAGAGAGTAAAGGTTGGCGAGAAGAGCTTTTT |
| 3-R | TCTAAAAAAGCTCTTCTCGCCAACCTTTACTCTCTTGAAGTAAAGGTTGGCGAGAAGAGCG |
| 4-F | TCCCCGCCGGAACCTTCCTTGCAAATTCAAGAGATTTGCAAGGAAGGTTCCGGCTTTTT |
| 4-R | TCTAAAAAAGCCGGAACCTTCCTTGCAAATCTCTTGAATTTGCAAGGAAGGTTCCGGCG |
| 5-F | TCCCCGCGTAAGAGTCTCCTCATTGGTTCAAGAGACCAATGAGGAGACTCTTACGCTTTTT |
| 5-R | TCTAAAAAAGCGTAAGAGTCTCCTCATTGGTCTCTTGAACCAATGAGGAGACTCTTACGCG |
| 6-F | TCCCCGCCCTTAGTGTGCGCATTATTCAAGAGATAATGCGCACACTAAGGGCTTTTT |
| 6-R | TCTAAAAAAGCCCTTAGTGTGCGCATTATCTCTTGAATAATGCGCACACTAAGGGCG |

In HEK293 cells cultured in vitro, Kir4.1 and the six shRNAs were co-expressed, respectively. FIG. 7 showed the results of Western blotting. The luciferase which is not expressed in animals was used as a negative control (NC). AAV2/5-H1-Luciferase-shRNA-gfaABC1D-EGFP was used as a control virus to express NC-shRNA. The results showed that the knock-off efficient of these 6 shRNAs were: Kir4.1-shRNA-1 > Kir4.1-shRNA-4 / Kir4.1-shRNA-5> Kir4.1-shRNA-2 > Kir4.1-shRNA-6 > Kir4.1-shRNA-3.

Kir4.1-shRNA-5 was packaged into AAV2/5 virus vector for Kir4.1 function down-regulation tests in rats by Taitool Bioscience of China.

FIG. 6A is a schematic diagram of an AAV virus vector used to down-regulate the expression level of Kir4.1 and a Kir4.1 dominant mutation of Kir4.1 function. FIG. 6B is the result of Western blot experiments, showing that Kir4.1-shRNA can effectively reduce Kir4.1 over-expressed on cells in HEK293 cells cultured in vitro.

The upper graph of FIG. 6C showed the time axis of down-regulated Kir4.1 function in rats for electrophysiological experiments and behavioral tests; the lower graph of immunofluorescence showed the expression of Kir4.1 dominant mutant virus in astrocytes of lateral habenula.

AAV-Kir4.1-shRNA virus-expressing rat LHb brain slices were subjected to electrophysiological recording. Resting membrane potentials of shRNA-expressing astrocyte were significantly depolarized; in neurons infected with the AAV-Kir4.1-shRNA vector, the RMPs did not differ from neighboring non-infected neurons, but are significantly more super-polarized than the neurons that express control shRNA (FIG. 6 D-G). There results suggested that knock-down of Kir4.1 in astrocytes had a global impact on RMPs of neighboring neurons. Bursting activity in LHb of cLH rats were completely eliminated by AAV-Kir4.1-shRNA viral infection (from 29% to 0%, FIG. 6H).

Behaviorally, infection of AAV-Kir4.1-shRNA had a pronounced effect on rescuing the depressive-like phenotypes of cLH rats in three depression paradigms: reducing the immobility time and increasing latency to immobility in FST (FIG. 6I), increasing bar pressing number in the LHT (FIG. 6J,K), and increasing the sucrose preference score in SPT (FIG. 6L).

To avoid an off-target effect of shRNA, a dominant-negative form of Kir4.1, dnKir4.1, containing a GYG to AAA point mutation at the channel pore which blocks K⁺ channels was also tested. The preparation of the Kir4.1 mutation construct and viral deliver system (namely AAV-dnKir4.1, or AAV5-gfaABC1D-dnKir4.1-2A-eGFP) was prepared as described in Example 1. Infection of AAVdnKir4.1 caused similarly strong anti-depression effects in cLH rats (FIG. 6I-L): reducing the immobility time and increasing latency to immobility in FST (FIG. 6I), increasing bar pressing number in the LHT (FIG. 6J,K), and increasing the sucrose preference score in SPT (FIG. 6L), while the mobility of mice were not changed (FIG. 8 B).

In addition, a dominant negative mutant of Kir4.1 was used to inhibit the function of LHb Kir4.1 channel in cLH rats. FIG. 6A shows the structure of the Kir4.1 dominant mutant virus AAV2/5-gfaABClD-Kir4.1dn-2A-EGFP (amino acid sequence 130-132 GYG in the channel pore region of the Kir4.1 sequence was mutated to be AAA to achieve a Kir4. 1 dominant negative mutation). Injection of AAV-dnKir4.1-EGFP virus in LHb significantly reversed the depressive phenotype of cLH rats: forced swimming immobility in rats was significantly reduced (as shown in FIG. 6I), and learned helplessness behavior increased significantly (as shown in FIGS. 6J and 6K), and the sugar and water preference also increased significantly (as shown in FIG. 6 L).

### Conclusions

After in-depth research, the inventors discover for the first time that Kir4.1 in astrocytes in the lateral habenula is a crucial regulator of depression. Using molecular, behavioral, and electrophysiological methods, it is found that Kir4.1 exists in the lateral phrenic nucleus in a way that surrounded the neuronal soma. Kir4.1 regulates the extracellular potassium balance, changes the firing characteristics of the lateral habenula neurons, and causes the lateral habenula nucleus to become overactive, and regulates the depression phenotype. The present inventors have also discovered and demonstrated a variety of agents that can block Kir4.1 activity in the lateral habenula, thereby providing methods and drugs for treating (inhibiting) depression by inhibiting Kir4.1 activity.

Unless otherwise indicated, the practice of the present disclosure will employ common technologies of organic chemistry, polymer chemistry, biotechnology, and the like. It is apparently that in addition to the above description and examples than as specifically described, the present disclosure can also be achieved in other ways. Other aspects within the scope of the disclosure and improvement of the present disclosure will be apparent to the ordinary skilled in the art. According to the teachings of the present disclosure, many modifications and variations are possible, and therefore it is within the scope of the present disclosure.

Unless otherwise indicated herein, the temperature unit "degrees" refers to Celsius degrees, namely °C.

## Claims

1. A pharmaceutical composition for use in treating depression in a subject, wherein the depression is **characterized by** abnormal burst firing in neurons of a lateral habenula of the subject, the pharmaceutical composition comprising an agent capable of suppressing expression, or inhibiting activity of Kir4.1 in astrocytes of the lateral habenula of the subject such that the abnormal burst firing of the neurons in the lateral habenula of the subject is suppressed.

2. The pharmaceutical composition for use of Claim 1, wherein the agent comprises RNA molecules capable of suppressing expression of Kir4.1 through RNA interference, wherein said RNA molecules target an mRNA of Kir4.1 whose nucleotide sequence is set forth in SEQ ID No. 7.

3. The pharmaceutical composition for use of Claim 2, wherein said RNA molecules target a nucleotide fragment within the mRNA of Kir4.1, the nucleotide fragment having one of sequences as set forth in:
| | |
|---|---|
| 5'-GGACGACCTTCATTGACAT-3' | (SEQ ID No. 1); |
| 5'-GCTACAAGCTTCTGCTCTTCT-3' | (SEQ ID No. 2); |
| 5'-CCGGAACCTTCCTTGCAAA-3' | (SEQ ID No. 4); |
| 5'-GCGTAAGAGTCTCCTCATTGG-3' | (SEQ ID No. 5); or |
| 5'-GCCCTTAGTGTGCGCATTA-3' | (SEQ ID No. 6). |

4. The pharmaceutical composition for use of Claim 2 or Claim 3, wherein said RNA molecules comprise small interfering RNA (siRNA) molecules, short hairpin RNA (shRNA) molecules, or micro-RNA (miRNA) molecules.

5. The pharmaceutical composition for use of Claim 1, wherein the agent comprises a mutant Kir4.1 protein or an encoding nucleotide sequence thereof, wherein said mutant Kir4.1 protein has a diminished or deactivated potassium channel activity, and has a mutation preferably within a channel pore region of Kir4.1 protein whose amino acid sequence as set forth in SEQ ID No. 8.

6. The pharmaceutical composition for use of Claim 5, wherein said mutation is a GYG to AAA mutation at positions 130-132 of the Kir4.1 protein.

7. The pharmaceutical composition for use of Claim 1, wherein the agent comprises a specific antibody against Kir4.1.

8. The pharmaceutical composition for use of Claim 1, wherein the agent comprises a small molecule agent capable of inhibiting activity of Kir4.1 in astrocytes in the lateral habenula of the subject.

## Patentansprüche

1. Pharmazeutische Zusammensetzung zur Verwendung bei der Behandlung von Depressionen bei einem Patienten, wobei die Depression durch abnormales Burst-Firing in Neuronen einer lateralen Habenula des Patienten gekennzeichnet ist, wobei die pharmazeutische Zusammensetzung ein Mittel umfasst, das die Expression oder die Aktivität von Kir4.1 in Astrozyten der lateralen Habenula des Patienten unterdrücken oder hemmen kann, so dass das abnormale Burst-Firing der Neuronen in der lateralen Habenula des Patienten unterdrückt wird.

2. Pharmazeutische Zusammensetzung zur Verwendung nach Anspruch 1, wobei das Mittel RNA-Moleküle umfasst, die die Expression von Kir4.1 durch RNA-Interferenz unterdrücken können, wobei die RNA-Moleküle auf eine mRNA von Kir4.1 abzielen, deren Nukleotidsequenz in SEQ ID No. 7 dargelegt ist.

3. Pharmazeutische Zusammensetzung zur Verwendung nach Anspruch 2, wobei die RNA-Moleküle auf ein Nukleotidfragment innerhalb der mRNA von Kir4.1ok d abzielen, wobei das Nukleotidfragment eine der folgenden Sequenzen aufweist:
5'-GGACGACCTTCATTGACAT-3' (SEQ ID No. 1);
5'-GCTACAAGCTTCTGCTCTTCT-3' (SEQ ID No. 2);
5'-CCGGAACCTTCCTTGCAAA-3' (SEQ ID No. 4);
5'-GCGTAAGAGTCTCCTCATTGG-3' (SEQ ID No. 5; oder
5'-GCCCTTAGTGTGCGCATTA-3' (SEQ ID No. 6).

4. Pharmazeutische Zusammensetzung zur Verwendung nach Anspruch 2 oder Anspruch 3, wobei die RNA-Moleküle kleine interferierende RNA (siRNA)-Moleküle, short-hairpin-RNA (shRNA)-Moleküle oder Mikro-RNA (miRNA)-Moleküle umfassen.

5. Pharmazeutische Zusammensetzung zur Verwendung nach Anspruch 1, wobei das Mittel ein mutiertes Kir4.1-Protein oder eine kodierende Nukleotidsequenz davon umfasst, wobei das mutierte Kir4.1-Protein eine verminderte oder deaktivierte Kaliumkanalaktivität aufweist und eine Mutation vorzugsweise innerhalb einer Kanalporenregion des Kir4.1-Proteins aufweist, dessen Aminosäuresequenz wie in SEQ ID No. 8 dargelegt ist.

6. Pharmazeutische Zusammensetzung zur Verwendung nach Anspruch 5, wobei die Mutation eine GYG-zu-AAA-Mutation an den Positionen 130-132 des Kir4.1-Proteins ist.

7. Pharmazeutische Zusammensetzung zur Verwendung nach Anspruch 1, wobei das Mittel einen spezifischen Antikörper gegen Kir4.1 umfasst.

8. Pharmazeutische Zusammensetzung zur Verwendung nach Anspruch 1, wobei das Mittel einen niedermolekularen Wirkstoff umfasst, der die Aktivität von Kir4.1 in Astrozyten in der lateralen Habenula des Patienten hemmen kann.

## Revendications

1. Composition pharmaceutique pour utilisation dans le traitement de la dépression chez un sujet, dans laquelle la dépression est **caractérisée par** une activation anormale dans les neurones d'une habénula latérale du sujet, la composition pharmaceutique comprenant un agent capable de supprimer l'expression, ou d'inhiber l'activité de Kir4.1 dans les astrocytes de l'habénula latérale du sujet de telle sorte que l'activation anormale des neurones dans l'habénula latérale du sujet est supprimée.

2. Composition pharmaceutique pour utilisation selon la revendication 1, dans laquelle l'agent comprend des molécules d'ARN capables de supprimer l'expression de Kir4.1 par l'intermédiaire d'une interférence par ARN, dans laquelle lesdites molécules d'ARN ciblent un ARNm de Kir4.1 dont la séquence nucléotidique est exposée dans SEQ ID No. 7.

3. Composition pharmaceutique pour utilisation selon la revendication 2, dans laquelle lesdites molécules d'ARN ciblent un fragment nucléotidique dans l'ARNm de Kir4.1, le fragment nucléotidique présentant une des séquences telles qu'exposées dans :
5'-GGACGACCTTCATTGACAT-3' (SEQ ID No. 1) ;
5'-GCTACAAGCTTCTGCTCTTCT-3' (SEQ ID No. 2) ;
5'-CCGGAACCTTCCTTGCAAA-3' (SEQ ID No. 4) ;
5'-GCGTAAGAGTCTCCTCATTGG-3' (SEQ ID No. 5) ; ou
5'-GCCCTTAGTGTGCGCATTA-3' (SEQ ID No. 6).

4. Composition pharmaceutique pour utilisation selon la revendication 2 ou revendication 3, dans laquelle lesdites molécules d'ARN comprennent des molécules d'ARN interférent court (ARNsi), des molécules d'ARN court en épingle à cheveux (shARN), ou des molécules de micro-ARN (miARN).

5. Composition pharmaceutique pour utilisation selon la revendication 1, dans laquelle l'agent comprend une protéine mutante Kir4.1 ou une séquence nucléotidique codant celle-ci, dans laquelle ladite protéine mutante Kir4.1 présente une activité de canal potassique diminuée ou désactivée, et présente une mutation de préférence dans une région de pore de canal de la protéine Kir4.1 dont la séquence en acides aminés telle qu'exposée dans SEQ ID No. 8.

6. Composition pharmaceutique pour utilisation selon la revendication 5, dans laquelle ladite mutation est une mutation de GYG en AAA aux positions 130-132 de la protéine Kir4.1.

7. Composition pharmaceutique pour utilisation selon la revendication 1, dans laquelle l'agent comprend un anticorps spécifique dirigé contre Kir4.1.

8. Composition pharmaceutique pour utilisation selon la revendication 1, dans laquelle l'agent comprend un agent à petite molécule capable d'inhiber l'activité de Kir4.1 dans les astrocytes dans l'habénula latérale du sujet.
